# FASCICULE DE BREVET EUROPEEN

(11) **EP 3 402 337 B1**
(45) Date de publication et mention de la délivrance du brevet: **07.08.2019**
(21) Numéro de dépôt: 17700227.6
(22) Date de dépôt: 10.01.2017
(51) Int. Cl.: A23C 9/13, A23C 9/152, A23C 21/06, A23C 21/10, A61K 33/06, A23L 33/16

(54) **CALCIUM LAITIER EN POUDRE DISPERSIBLE, SON PROCÉDÉ DE PRÉPARATION ET SON UTILISATION POUR ENRICHIR EN CALCIUM DES ALIMENTS**
MILCHCALCIUM IN DISPERGIERBARER PULVERFORM, VERFAHREN ZUR HERSTELLUNG DAVON UND VERWENDUNG DAVON ZUR ANREICHERUNG VON LEBENSMITTELN MIT CALCIUM
MILK CALCIUM IN DISPERSIBLE POWDER FORM, METHOD OF PREPARATION THEREOF AND USE OF SAME TO ENRICH FOOD WITH CALCIUM

(30) Priorité: 11.01.2016 FR 1650206
(43) Date de publication de la demande: 21.11.2018
(73) Titulaire: Compagnie Laitiere Europeenne, 50890 Conde-sur-Vire (FR)
(72) Inventeur: LAROCHE, Christophe, 35300 Fougeres (FR); KERRIOU, Liliane, 35340 Liffre (FR); KERVINIO, Audrey, 35340 Liffre (FR)
(74) Mandataire: Gevers & Orès
(86) Numéro de dépôt international: PCT/EP2017/050408
(87) Numéro de publication internationale: WO 2017/121726

(56) Documents cités:
- EP-A2- 1 147 712
- WO-A1-2014/163975
- WO-A1-2015/148384
- WO-A2-2009/004566
- JP-A- 2005 073 695
- US-A- 3 943 264
- US-A1- 2003 118 662

## Description

La présente invention se rapporte au domaine des ingrédients alimentaires laitiers. Elle vise plus spécifiquement une composition de calcium laitier en poudre dispersible destinée à être ajoutée à des aliments dans le but d'en augmenter la teneur en calcium.

Il est connu de supplémenter en calcium des aliments ou des boissons pour leur apporter un intérêt nutritionnel accru. Cette supplémentation peut être réalisée par l'ajout de calcium de sources diverses : d'origine marine comme décrit dans l'US 2008/0299279 ; de synthèse comme les sels de calcium organiques alimentaires -voir notamment le WO 2009/004566 qui propose une poudre de lactosérum enrichie avec de tels sels de calcium- ou encore d'origine laitière, comme par exemple le sérum de lait de faible pH et concentré en protéines et calcium laitiers destiné à enrichir des boissons décrit dans l'US2008/0063765 ou la composition de calcium de lait décrite dans l'EP 1 147 712.

Quelle que soit son origine, la supplémentation en calcium est susceptible d'altérer les propriétés organoleptiques de l'aliment ou de la boisson supplémenté : précipitation et/ou sédimentation du calcium, gout métallique, sensation en bouche pâteuse ou crayeuse, voire de grains de sable.

La Demanderesse s'est donné comme objectif la mise au point d'un produit de supplémentation en calcium qui ait une origine exclusivement laitière et qui n'altère pas les propriétés organoleptiques de l'aliment ou de la boisson auquel il est ajouté. Elle est parvenue à ce résultat avec un produit qui présente une excellente dispersibilité et qui contient une teneur en calcium comprise entre 7 et 11% en masse, ce qui est bien supérieur à la teneur décrite dans l'EP 1 147 712 dans lequel les auteurs mentionnent une baisse inacceptable de la dispersibilité du produit de supplémentation en calcium lorsque la teneur en calcium dépasse 5% en masse.

La présente invention se rapporte ainsi à un calcium laitier dispersible en poudre, à son procédé de préparation et à son utilisation pour la supplémentation en calcium d'un aliment.

Le calcium laitier selon l'invention est dit dispersible dans la mesure où, lorsqu'on l'introduit dans de l'eau sous agitation, il se disperse dans l'eau et reste en suspension sans dépôt observable comme illustré à l'exemple 2.

Selon un premier objet, la présente invention se rapporte à un procédé de préparation d'un calcium laitier dispersible en poudre caractérisé en ce qu'il comprend les étapes suivantes :
a. le mélange d'une poudre de phosphate de calcium laitier micronisée et d'un lactosérum de fromagerie, déminéralisé ou non, ayant un pH compris entre 6 et 7.5, éventuellement concentré ;
b. le séchage du mélange de l'étape a.

Selon un mode de réalisation préféré, la poudre de phosphate de calcium laitier micronisée comprend entre 68 et 85% de cendres, dont entre 20 et 35% en masse de calcium et entre 9 à 15% en masse de phosphore, 0,1 à 15% en masse de lactose et 10 à 15% en masse de protéines laitières.

La poudre de phosphate de calcium laitier micronisée, utilisée en tant que matière première pour réaliser le calcium laitier dispersible, a une granulométrie moyenne (diamètre moyen) de 1 à 5 µm et une surface spécifique de 41 à 120 m²/g. Lorsqu'elle est soumise à un isotherme de sorption, elle reprend en eau de 10 à 25% en masse.

Par lactosérum de fromagerie, déminéralisé ou non, ayant un pH compris entre 6 et 7.5, on entend un lactosérum issu de la fabrication de fromage mais également tout produit reconstitué pouvant être obtenu par mélange d'un perméat et de protéines laitières ; la préparation d'un tel lactosérum reconstitué est connue de l'homme du métier.

La sédimentation de cette poudre de phosphate de calcium laitier micronisée dans de l'eau, du lait ou autre liquide laitier est réalisée en moins d'une heure et en général en 15 min selon la méthode décrite dans l'exemple 2 (éprouvettes A).

Le mélange de l'étape a peut être réalisé par tout moyen connu de l'homme du métier.

Il en est de même du séchage de l'étape b ; de préférence, il est effectué en tour de séchage par atomisation.

Le procédé selon l'invention peut en outre comprendre une étape c additionnelle de micronisation ; cette étape c peut être réalisée à l'aide d'un microniseur.

La présente invention se rapporte également à la poudre susceptible d'être obtenue par le procédé selon l'invention, c'est-à-dire à un calcium laitier dispersible en poudre comprenant entre 7 et 11% en masse de calcium, entre 5 et 15% en masse de protéines laitières et entre 50 et 70% en masse de lactose.

Le calcium laitier dispersible en poudre selon l'invention se présente sous la forme d'une poudre homogène.

La granulométrie de la poudre est comprise entre 1 et 10 µm, de préférence, elle est de l'ordre de 1 µm si la poudre est micronisée, c'est-à-dire issue du procédé selon l'invention comprenant une étape c de micronisation et sa surface spécifique est comprise entre 10 et 100m²/g. Alternativement, si le procédé selon l'invention ne comprend pas d'étape c de micronisation, la poudre a une granulométrie comprise entre 15 et 150 µm, généralement de l'ordre de 150 µm et sa surface spécifique est comprise entre 0,2 et 15 m²/g.

Lorsqu'elle est soumise à un isotherme de sorption, elle reprend en eau de 5% à 15% en masse.

Le calcium laitier dispersible en poudre selon l'invention, lorsqu'il est mis en solution dans de l'eau, du lait ou autre produit laitier liquide type yaourt à boire, ne sédimente pas, notamment dans un test de sédimentation tel que décrit dans l'exemple 2 (éprouvettes B).

La présente invention se rapporte encore à l'utilisation du calcium laitier dispersible en poudre pour la préparation d'aliment, de préférence de produit laitier tel que des produits laitiers UHT et frais, enrichi en calcium.

Le calcium laitier dispersible en poudre selon l'invention permet d'enrichir les aliments en calcium, par exemple les produits laitiers, sans apporter de défaut de goût, ni de sensation crayeuse et granuleuse et sans dépôt (sédimentation).

L'homme du métier saura ajuster la quantité de poudre à ajouter à l'aliment en fonction de la teneur finale en calcium souhaitée qu'il pourra notamment déterminer au regard des apports journaliers recommandés en calcium.

Dans certaines applications, par exemple dans le domaine des produits laitiers, une augmentation de la viscosité du produit est observée lors de l'ajout du calcium laitier dispersible en poudre selon l'invention (voir la partie expérimentale).

### Figures

La Figure 1 représente des éprouvettes contenant du lait coloré au bleu de méthylène et mélangé à du phosphate de calcium classique (éprouvettes A) ou du calcium laitier en poudre dispersible selon l'invention obtenu selon le procédé de l'exemple 1 (éprouvettes B) photographiées après 15, 30 et 60 minutes comme décrit dans l'exemple 2.
La Figure 2 est un graphe représentant la viscosité de différents yaourts à boire préparés respectivement sans calcium ajouté, avec du phosphate de calcium classique et avec du calcium laitier en poudre dispersible selon l'invention comme décrit à l'exemple 3.

### Exemples

### Exemple 1 - préparation d'un calcium laitier en poudre dispersible selon l'invention

Un calcium laitier dispersible en poudre selon l'invention est préparé selon le protocole suivant :
- concentration et pasteurisation du lactosérum doux de fromagerie ;
- ajout dans ce lactosérum d'une poudre de phosphate de calcium laitier micronisé contenant 28% en masse de calcium, 1% en masse de lactose et 12% en masse de protéines laitières ;
- mélange du phosphate de calcium et du lactosérum ; et
- séchage du mélange.

### Exemple 2 - caractérisation du calcium laitier en poudre dispersible selon l'exemple 1

### Mode opératoire

- Peser 3g de calcium laitier en poudre dispersible selon l'exemple 1 d'une part et de phosphate de calcium classique (Composition : calcium 26% en masse, phosphore 14 % en masse, cendres 75% en masse, lactose 9% en masse, protéines 3 % en masse) d'autre part ;
- Introduire ces 3g dans 50 mL de lait écrémé coloré avec du bleu de méthylène ;
- Agiter pendant 15 mn (agitation magnétique) ;
- Transvaser dans des éprouvettes de 50 mL ; les éprouvettes A contiennent le lait mélangé au phosphate de calcium classique et les éprouvettes B contiennent le lait mélangé au calcium laitier en poudre dispersible selon l'invention obtenu selon le procédé de l'exemple 1;
- Démarrer le chronomètre.

Les éprouvettes sont ensuite photographiées à différents temps pour évaluer l'apparition éventuelle d'un dépôt.

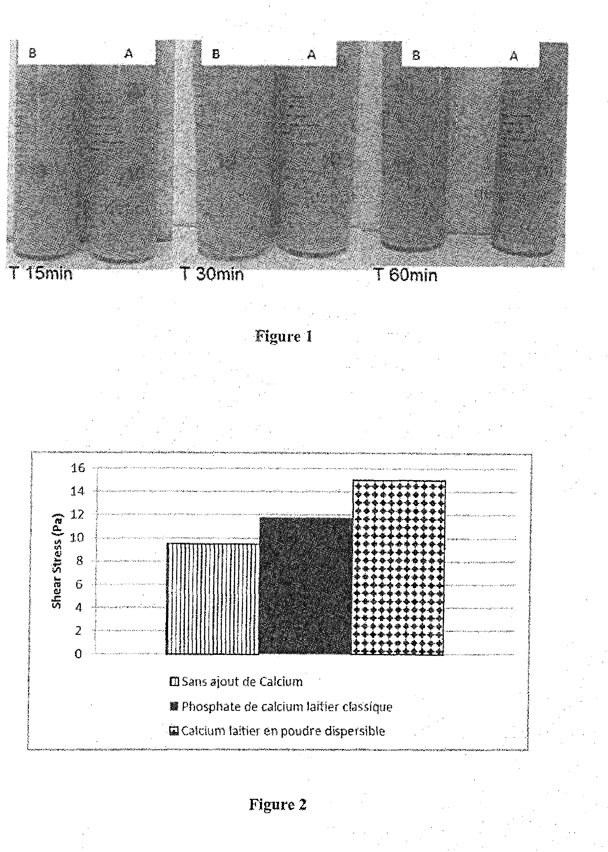

Les photographies de la Figure 1 montrent que :
- dans les éprouvettes A contenant le phosphate de calcium classique, un dépôt est observé dès 15 min de repos de l'éprouvette ;
- dans les éprouvettes B contenant le calcium laitier en poudre dispersible selon l'exemple 1, aucun dépôt observé après 1 heure de repos de l'éprouvette.

### Exemple 3 - utilisation en yaourt à boire du calcium laitier en poudre dispersible selon l'exemple 1

### Préparation des yaourts à boire :

Trois batchs de yaourts à boire sont préparés selon le procédé suivant :
- pesée du lait écrémé (1,5 litre) ;
- optionnellement, ajout de 8g phosphate de calcium classique ou de 23 g de calcium laitier en poudre dispersible selon l'invention (exemple 1) ;
- pasteurisation du le mélange précédent ;
- ensemencement de ce mélange avec 0,08 g de ferments lactiques ;
- fermentation jusqu'à obtention du pH cible (pH 4.5) ;
- refroidissement des yaourts à 10°C et conditionnement dans des contenants adaptés ;

### Caractérisation de la viscosité des yaourts obtenus

La viscosité des yaourts à boire est mesurée avec un viscosimètre.

Les histogrammes de la Figure 2 illustrent la viscosité de chacun de ces yaourts à boire ; ils montrent en particulier une viscosité accrue lorsque le calcium laitier en poudre dispersible selon l'invention est utilisé pour la fabrication du yaourt à boire.

### Evaluation sensorielle des yaourts obtenus

L'ajout du calcium laitier en poudre dispersible selon l'invention n'entraîne pas de défaut de goût ou dépôt dans le yaourt à boire.

## Revendications

1. Procédé de préparation d'un calcium laitier dispersible en poudre **caractérisé en ce qu'**il comprend les étapes suivantes :
a. le mélange d'une poudre de phosphate de calcium laitier micronisée et d'un lactosérum de fromagerie, déminéralisé ou non, ayant un pH compris entre 6 et 7.5, éventuellement concentré ;
b. le séchage du mélange de l'étape a ;
**caractérisé en ce que** la poudre de phosphate de calcium laitier micronisée comprend entre 68 et 85% de cendres, dont entre 20 et 35% en masse de calcium et entre 9 à 15% en masse de phosphore, 0,1 à 15% en masse de lactose et 10 à 15% en masse de protéines laitières et **en ce que** la poudre de phosphate de calcium laitier micronisée a une granulométrie moyenne de 1 à 5 µm et une surface spécifique de 41 à 120 m²/g.

2. Procédé selon la revendication 1, **caractérisé en ce que** l'étape b de séchage est réalisée en tour de séchage par atomisation.

3. Procédé selon la revendication 1 ou 2, **caractérisé en ce qu'**il comprend une étape c additionnelle de micronisation de la poudre obtenue à l'étape b.

4. Calcium laitier dispersible en poudre susceptible d'être obtenu selon le procédé de l'une quelconque des revendications 1 à 3, **caractérisé en ce qu'**il comprend entre 7 et 11% en masse de calcium, entre 5 et 15% en masse de protéines laitières et entre 50 et 70% en masse de lactose.

5. Calcium laitier dispersible en poudre selon la revendication 4, **caractérisé en ce qu'**il est sous la forme d'une poudre homogène.

6. Calcium laitier dispersible en poudre selon la revendication 4 ou 5, **caractérisé en ce que** sa granulométrie est comprise entre 1 et 10 µm et sa surface spécifique est comprise entre 10 et 100 m²/g s'il est micronisé, c'est-à-dire issu du procédé selon la revendication 3, ou sa granulométrie est comprise entre 15 et 150 µm et sa surface spécifique est comprise entre 0,2 et 15 m²/g s'il n'est pas micronisé.

7. Utilisation du calcium laitier dispersible en poudre selon l'une quelconque des revendications 4 à 6 pour la préparation d'aliment enrichi en calcium.

8. Utilisation du calcium laitier dispersible en poudre selon la revendication 7, **caractérisée en ce que** l'aliment enrichi en calcium est un produit laitier.

9. Utilisation du calcium laitier dispersible en poudre selon la revendication 7 ou la revendication 8, **caractérisée en ce que** ledit calcium laitier dispersible en poudre ne sédimente pas dans ledit aliment enrichi.

## Patentansprüche

1. Verfahren zur Herstellung eines Milchcalciums in dispergierbarer Pulverform, **dadurch gekennzeichnet, dass** es die folgenden Schritte umfasst:
a. Mischen eines mikronisierten Milchcalciumphosphatpulvers und einer demineralisierten oder nicht demineralisierten Molke zur Käseherstellung, die einen pH-Wert aufweist, der zwischen 6 und 7,5 enthalten ist, eventuell konzentriert;
b. Trocknen der Mischung von Schritt a;
**dadurch gekennzeichnet, dass** das mikronisierte Milchcalciumphosphatpulver zwischen 68 und 85 % Asche umfasst, davon zwischen 20 und 35 Massenprozent Calcium und zwischen 9 bis 15 Massenprozent Phosphor, 0,1 bis 15 Massenprozent Lactose und 10 bis 15 Massenprozent Milchproteine, und dadurch, dass das mikronisierte Milchcalciumphosphatpulver eine mittlere Granulometrie von 1 bis 5 µm und eine spezifische Oberfläche de 41 bis 120 m²/g aufweist.

2. Verfahren nach Anspruch 1, **dadurch gekennzeichnet, dass** der Schritt b des Trocknens in einem Trocknungsturm durch Zerstäubung erfolgt.

3. Verfahren nach Anspruch 1 oder 2, **dadurch gekennzeichnet, dass** es einen zusätzlichen Schritt c des Mikronisierens des in Schritt b erhaltenen Pulvers umfasst.

4. Milchcalcium in dispergierbarer Pulverform, das nach dem Verfahren eines der Ansprüche 1 bis 3 erhalten werden kann, **dadurch gekennzeichnet, dass** es zwischen 7 und 11 Massenprozent Calcium, zwischen 10 und 15 Massenprozent Milchproteine und zwischen 50 und 70 Massenprozent Lactose umfasst.

5. Milchcalcium in dispergierbarer Pulverform nach Anspruch 4, **dadurch gekennzeichnet, dass** es in Form eines homogenen Pulvers ist.

6. Milchcalcium in dispergierbarer Pulverform nach Anspruch 4 oder 5, **dadurch gekennzeichnet, dass** seine Granulometrie zwischen 1 und 10 µm enthalten ist und seine spezifische Oberfläche zwischen 10 und 100 m²/g enthalten ist, wenn es mikronisiert ist, d. h. aus dem Verfahren nach Anspruch 3 stammt, oder seine Granulometrie zwischen 15 und 150 µm enthalten ist und seine spezifische Oberfläche zwischen 0,2 und 15 m²/g enthalten ist, wenn es nicht mikronisiert ist.

7. Verwendung des Milchcalciums in dispergierbarer Pulverform nach einem der Ansprüche 4 bis 6 zur Herstellung eines mit Calcium angereicherten Lebensmittels.

8. Verwendung des Milchcalciums in dispergierbarer Pulverform nach Anspruch 7, **dadurch gekennzeichnet, dass** das mit Calcium angereicherte Lebensmittel ein Milchprodukt ist.

9. Verwendung des Milchcalciums in dispergierbarer Pulverform nach Anspruch 7 oder Anspruch 8, **dadurch gekennzeichnet, dass** sich das Milchcalcium in dispergierbarer Pulverform in dem angereicherten Lebensmittel nicht absetzt.

## Claims

1. Method for preparing a dispersible dairy calcium in powder form, **characterised in that** it comprises the following steps:
a) mixing a micronised dairy calcium phosphate powder and a cheese-making whey, demineralised or not, having a pH of between 6 and 7.5, optionally concentrated;
b) drying the mixture of step a;
**characterised in that** the micronised dairy calcium phosphate powder comprises between 68 and 85% ash, including between 20 and 35% by mass calcium and between 9 and 15% by mass phosphorus, 0.1 to 15% by mass lactose and 10 to 15% by mass dairy proteins, and **in that** the micronised dairy calcium phosphate powder has a mean particle size of 1 to 5 µm and a specific surface area of 41 to 120 m²/g.

2. Method according to claim 1, **characterised in that** the drying step b is carried out in a drying tower by atomisation.

3. Method according to claim 1 or 2, **characterised in that** it comprises an additional step c of micronisation of the powder obtained at step b.

4. Dispersible dairy calcium in powder form obtainable according to the method of any of claims 1 to 3, **characterised in that** it comprises between 7 and 11% by mass calcium, between 5 and 15% by mass dairy proteins and between 50 and 70% by mass lactose.

5. Dispersible dairy calcium in powder form according to claim 4, **characterised in that** it is in the form of a homogeneous powder.

6. Dispersible dairy calcium in powder form according to claim 4 or 5, **characterised in that** its particle size is between 1 and 10 µm and its specific surface area is between 10 and 100 m²/g if it is micronised, that is to say resulting from the method according to claim 3, or its particle size is between 15 and 150 µm and its specific surface area is between 0.2 and 15 m²/g if it is not micronised.

7. Use of the dispersible dairy calcium in powder form according to any of claims 4 to 6 for preparing a calcium-enriched food.

8. Use of the dispersible dairy calcium in powder form according to claim 7, **characterised in that** the calcium-enriched food is a dairy product.

9. Use of the dispersible dairy calcium in powder form according to claim 7 or claim 8, **characterised in that** said dispersible dairy calcium in powder form does not sediment in said enriched food.
